# EUROPEAN PATENT APPLICATION

(11) **EP 2 843 381 A1**
(43) Date of publication of application: **04.03.2015**
(21) Application number: 14181832.8
(22) Date of filing: 21.08.2014
(51) Int. Cl.: G01J 3/433, G01N 29/24, G01N 21/17, G01N 33/28, G01N 21/3577, G01N 21/359

(54) **Method and system for detecting components in a fluid**

(30) Priority: 29.08.2013 IN CH38392013
(71) Applicant: General Electric Company, Schenectady, New York 12345 (US)
(72) Inventor: Kavoori Sethumadhavan, Nagapriya, 560066 Bangalore (IN); Maity, Sandip, 560066 Bangalore (IN); Choudhury, Niloy, 560066 Bangalore (IN)
(74) Representative: Illingworth-Law, William Illingworth

(57) **Abstract**

A system 200 and a method for detecting components 230 in a sample fluid 232 includes a first chamber 218 having a sample fluid 232 and a second chamber 216 coupled to the first chamber 218, wherein the second chamber 216 has a reference fluid 234. The system 200 includes a modulated light source 202 for emitting a modulated light beam 220 to the sample fluid 232 and the reference fluid 234, to generate a first acoustic signal 238 in the first chamber 218 and a second acoustic signal 240 in the second chamber 260. The system 200 further includes a pressure sensor 236 disposed between the first chamber 218 and the second chamber 216, for detecting a difference between the first acoustic signal 238 and the second acoustic signal 240. The system includes a processor based module 228 communicatively coupled to the pressure sensor 236 and configured to receive a signal representative of the difference and determine at least one of a component 230 and the concentration of the component 230 in the sample fluid 232.

## Description

The subject matter disclosed herein generally relates to detection systems and in particular, to a detection system for measuring at least one of a component and concentration of the component in a fluid using a photo acoustic spectroscopy (PAS) technique.

Electrical equipment, such as transformers, use fluids having good thermal and insulation properties to encapsulate components of the electrical equipment in a containment vessel for enabling dissipation of heat generated from a coil. The fluid may be an oil such as castor oil, mineral oil, synthetic oil such as chlorinated diphenyl silicone oil, or the like.

Failure of electrical equipment or components of the electrical equipment, such as coils of a transformer, may result in disruption of operation. Monitoring of the electrical equipment to predict potential failures through detection of incipient faults is desirable. A known method of monitoring the electrical equipment involves analysis of various parameters of the fluid that circulates about the equipment.

Presence of total combustible gas (TCG) in the fluid is known to provide information about operating state of the electrical equipment immersed in the fluid. To enable early detection of faults, the dissolved gases within the fluid are analyzed. Presence of gaseous components such as carbon monoxide, carbon dioxide, or the like and their concentrations, for example may be indicative of thermal aging of the equipment. Similarly, gaseous components such as hydrogen, hydrocarbons, or the like may be indicative of a dielectric breakdown among other faults.

Known methods for analyzing dissolved gases such as Gas Chromatography (GC), Optical Spectroscopy, and Photo Acoustic Spectroscopy (PAS), require the extraction of gases from the fluid. The known extraction techniques such as vacuum extraction, and head space extraction methods suffer from drawbacks such as repeatability issues and increased complexity.

There is a need for an enhanced technique to measure at least one of a component and concentration of the component in a fluid used in electrical equipment.

In accordance with one aspect, a system for detecting components in a sample fluid is disclosed. The system includes a first chamber having the sample fluid and a second chamber coupled to the first chamber, wherein the second chamber has a reference fluid. The system also includes a modulated light source for emitting a modulated light beam to the sample fluid and the reference fluid, to generate a first acoustic signal in the first chamber and a second acoustic signal in the second chamber. The system further includes a pressure sensor disposed between the first chamber and the second chamber, for detecting a difference between the first acoustic signal and the second acoustic signal. The system includes a processor based module communicatively coupled to the pressure sensor and configured to receive a signal representative of the difference from the pressure sensor and determine at least one of a component and a concentration of the component in the sample fluid based on the signal representative of the difference.

In accordance with another aspect, a method for detecting components in a sample fluid is disclosed. The method includes emitting a modulated light beam to the sample fluid in a first chamber and a reference fluid in a second chamber, wherein the second chamber is coupled to the first chamber. The method also includes generating a first acoustic signal in the first chamber and a second acoustic signal in the second chamber, in response to the emitted modulated light beam. The method further includes detecting a difference between the first acoustic signal and the second acoustic signal via a pressure sensor disposed between the first chamber and the second chamber. The method also includes transmitting a signal representative of the difference from the pressure sensor to a processor based module and determining at least one of a component and a concentration of the component in the sample fluid via the processor based module, based on the signal representative of the difference.

These and other features and aspects of embodiments of the present invention will become better understood when the following detailed description is read with reference to the accompanying drawings in which like characters represent like parts throughout the drawings, wherein:
FIG. 1 is a diagrammatic illustration of an electrical utility monitored in accordance with an exemplary embodiment;
FIG. 2 illustrates a detection system in accordance with an exemplary embodiment;
FIG. 3 illustrates relative positions of a center of a pressure wave front and a pressure sensor in accordance with an exemplary embodiment;
FIG. 4 illustrates a graph showing variation of pressure waveform detected by a pressure sensor in accordance with an exemplary embodiment;
FIG. 5 illustrates a side-by-side arrangement of a detection system in accordance with an exemplary embodiment;
FIG. 6 illustrates a top view of the side-by-side arrangement of a detection system in accordance with an exemplary embodiment;
FIG. 7 illustrates a concentric arrangement of a detection system in accordance with an exemplary embodiment;
FIG. 8 illustrates of a detection system having a single enclosure partitioned into two chambers;
FIG. 9 a graph representative of variation in amplitude of a photo acoustic pressure wave corresponding to a reference liquid in accordance with an exemplary embodiment;
FIG. 10 is a graph representative of variation in amplitude of a photo acoustic pressure wave corresponding to a component in a sample fluid in accordance with an exemplary embodiment;
FIG. 11 is a graph representative of an absorption spectrum of a reference liquid in accordance with an exemplary embodiment;
FIG. 12 is a graph representative of an absorption spectra corresponding to a plurality of gaseous components in accordance with an exemplary embodiment; and
FIG. 13 is a flow chart illustrating exemplary steps involved in detecting a component dissolved in a liquid in accordance with an exemplary embodiment.

Embodiments relate to systems and methods for detecting the presence of a component in a fluid using a spectroscopic method. Specifically, a technical effect is that in certain embodiments, the composition and concentration of a dissolved gas in a liquid is determined using photo acoustic spectroscopy (PAS) technique. A light beam from a modulated light source is emitted on a sample fluid in a first chamber and a reference fluid in a second chamber coupled to the first chamber. A pressure sensor disposed between the first chamber and the second chamber, measures a difference between a first acoustic signal generated in the first chamber and a second acoustic signal generated in the second chamber. A processor based module which is communicatively coupled to the pressure sensor, receives a signal representative of the difference from the pressure sensor and determines at least one of a component and a concentration of the component in the sample fluid based on the signal representative of the difference.

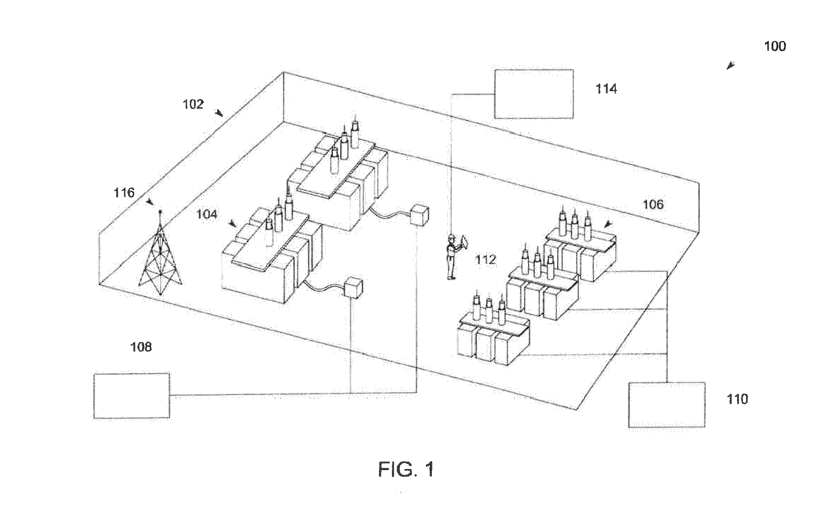

FIG. 1 illustrates an electrical utility 100 incorporating an exemplary system for inspection of equipment disclosed herein. The electrical utility 100 has an electrical infrastructure 102 having equipment 104, 106 which are to be inspected. In the illustrated embodiment, the equipment 104,106 are transformers. The equipment 104 is monitored by an exemplary inspection system 108 and the equipment 106 is monitored by another exemplary inspection system 110. Further, a portable diagnostic subsystem 114 may be used by a mobile operator 112 for quick and accurate diagnostics of the health of the equipment 104, 106. Additionally, the electrical utility 100 may also be equipped with a remote monitoring and diagnostic subsystem 116 for providing continuous asset monitoring capability. The remote monitoring in this example includes online fault monitoring and trending of faults to predict failure of the equipment 104, 106. It should be noted herein that the illustrated electrical utility 100 should not be construed as a limitation. In other words, the exemplary inspection systems are applicable for other applications in which there is a requirement for detecting the presence of a component in a fluid.

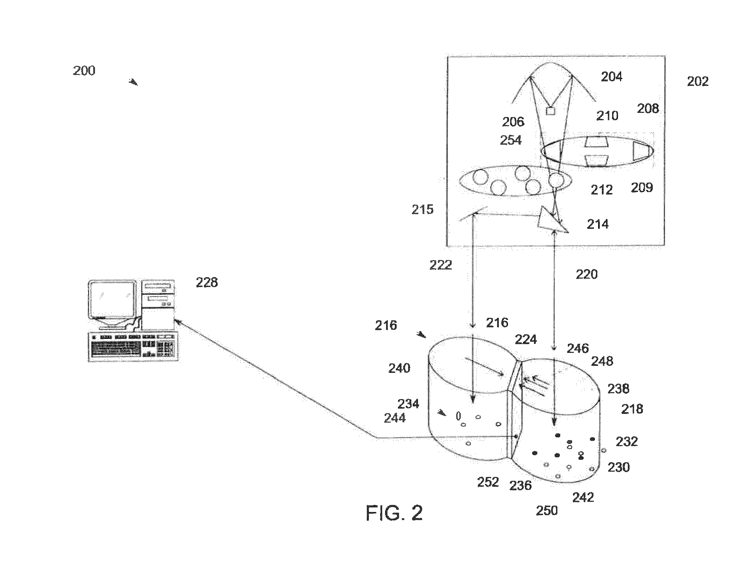

FIG. 2 illustrates a detection system 200 used in at least one of the inspection systems 108, 110 in accordance with an exemplary embodiment. The system 200 includes a modulated light source 202 for emitting a modulated light beam 220 to a sample fluid 232 filled in a first chamber 218 to generate a "first acoustic signal" 238. The "first acoustic signal" 238 referenced herein is a pressure signal generated by fluctuations in temperature of the sample fluid 232, wherein the fluctuations in temperature are caused by the modulated light beam 220. The modulated light source 202 further emits a modulated light beam 222 to a reference fluid 234 filled in a second chamber 216, to generate a "second acoustic signal" 240. The "second acoustic signal" 240 referenced herein is a pressure signal generated by fluctuations in temperature in the reference fluid 234, wherein the fluctuations in temperature are caused by the modulated light beam 222. In the illustrated embodiment, there is a single light source 206 that employs optics 204, 214, 215 to generate the modulated light beams 220, 222. In an alternate embodiment, the modulated light source 202 includes a first modulated light source for emitting modulated light beam 220 and a second modulated light source for emitting modulated light beam 222.

In the illustrated embodiment, the modulated light source 202 includes a light source 206 for generating the light beam 254 and a modulator device 208 for generating modulated light beams 220, 222. In one embodiment, the light source 206 is a laser light source. In alternate embodiments, the light source 206 may be a broad band light source, a tunable diode (TD) laser, or a quantum cascade laser source. In the illustrated embodiment, the modulated light source 202 includes a reflector 204 used for reflecting the light beam 254 from the light source 206.

The modulator device 208 modulates the light beam 254 from the light source 206 via the reflector 204 by controlling at least one of an intensity of the light beam, a wavelength of the light beam, parameters of the light source 206 and/or characteristics of the reflector 204. In the illustrated embodiment, the modulator device 208 includes a rotatable disc 209 with a plurality of slots 210. The rotatable disc 209 is used for generating a modulated light beam in the form of light pulses via the slots 210. In alternate embodiments, the modulator device 208 may be used to modulate the intensity of the light beam 254 from the light source 206 by other suitable techniques. In one specific embodiment, the modulator device 208 may be used to modulate the wavelength of the light beam. In some embodiments, the modulator device 208 may also be a part of the light source 206. In certain embodiments, the light beam 254 from the light source 206 may be modulated by varying one of more parameters of the light source 206. In one embodiment, the temperature of the light source 206 may be modified to generate a modulated light beam. In another embodiment, the current used for powering the light source 206, may be varied to generate a modulated light beam. The modulated light beam has a range of wavelength suitable for detecting the presence of one or more components in the fluid.

In the illustrated embodiment, the modulated light source 202 includes a reflector 204 and the light source 206. The light source 206 generates a light beam that strikes the reflector 204 and produces a reflected light beam 254. the modulated light source 202 also includes a filter 212 for filtering the light beam 254, corresponding to the required wavelength. In a specific embodiment, the filter 212 includes a plurality of filter lenses having different wavelengths. Further, a beam splitter 214 and a reflector 215 are used to generate modulated light beams 220, 222 from a filtered output of the filter 212. In the illustrated embodiment, two modulated light beams 220, 222 are generated from a single modulated light source 202. In another embodiment, two laser sources may be used to generate two modulated light beams.

In the illustrated embodiment, the sample fluid 232 includes a component 230 dissolved in a sample liquid 242 and the reference fluid 234 includes a reference liquid 244. In another embodiment, the sample fluid may be a suspension having a component in the sample liquid. In an exemplary embodiment, the sample liquid 242 and the reference liquid 244 may be an insulation oil used in the components to be inspected, for example a transformer. The component 230 may be at least one of gaseous components such as acetylene, hydrogen, methane, ethane, ethylene, carbon dioxide, carbon monoxide, moisture, or the like. In one embodiment, the constituents of the sample liquid 242 may be exactly same as the constituents of the reference liquid 244. In another embodiment, the constituents of the sample liquid 242 may be substantially same as the constituents of the reference liquid 244.

The first acoustic signal 238 generated in the first chamber 218, includes a third acoustic signal 246 generated due to the presence of the sample liquid 242 and a fourth acoustic signal 248 generated due to the presence of the component 230. The "third acoustic signal" 246 referenced herein is a pressure signal generated due to fluctuations in temperature of the sample liquid 242 generated by the modulated light beam 220. The "fourth acoustic signal" 248 is a pressure signal generated due to fluctuations in temperature of the component 230 generated by the modulated light beam 220. The "second acoustic signal" 240 referred herein is a pressure signal generated due to fluctuations in temperature of the reference liquid 244 generated by the modulated light beam 222. In the illustrated embodiment, a light absorption spectrum corresponding to the sample liquid 242 is the same as a light absorption spectrum corresponding to the reference liquid 244. A light absorption spectrum corresponding to the component 230 is different from a light absorption spectrum corresponding to the sample liquid 242.

In the illustrated embodiment, the first chamber 218 is coupled to the second chamber 216 and separated from each other by a diaphragm 224. A pressure sensor 236 is disposed in the diaphragm 224 between the first chamber 218 and the second chamber 216. The first acoustic signal 238 is transmitted to a first side 250 of the pressure sensor 236 and the second acoustic signal 240 is transmitted to a second side 252 opposite to the first side 250 of the pressure sensor 236. The third acoustic signal 246 corresponding to the sample liquid 242 is transmitted to the first side 250 of the pressure sensor 236. In an embodiment where the constituents of the sample liquid 242 are the same as the constituents of the reference liquid 244, the third acoustic signal 246 neutralizes or cancels the second acoustic signal 240 corresponding to the reference liquid 244. In another embodiment where the constituents of the sample liquid 242 are substantially the same as the constituents of the reference liquid 244, the third acoustic signal 246 substantially neutralizes the second acoustic signal 240. In one example, the third acoustic signal 246 is reduced by about 80dB. In other embodiments, the reduction would be in the range of about 40 to 100dB.

The pressure sensor 236 detects a difference between the first acoustic signal 238 and the second acoustic signal 240 and generates a signal representative of the difference. In other words, the pressure sensor 236 detects the pressure signal which is representative of the component 230.

In one embodiment, the pressure sensor 236 is positioned in the diaphragm 224. In an alternate embodiment, the diaphragm 224 may be the pressure sensor separating the first chamber 218 from the second chamber 216. In one embodiment, the pressure sensor 236 is a piezo-based pressure sensor. In such an embodiment, the pressure sensor 236 may employ a piezo-electric effect or a piezo-resistance effect to detect the difference between the first acoustic signal 238 and the second acoustic signal 240. In certain other embodiments, the pressure sensor 236 may be a cantilever-based pressure sensor or a membrane based pressure sensor, a microphone, a hydrophone or a capacitance based sensor.

A processor-based module 228 is communicatively coupled to the pressure sensor 236, and configured to receive the signal representative of the difference between the first acoustic signal 238 and the second acoustic signal 240 from the pressure sensor 236. The processor-based module 228 is also configured to determine at least one component and a concentration of the component 230 in the sample fluid 232 based on the signal representative of the difference between the first acoustic signal 238 and the second acoustic signal 240.

The processor-based module 228 may include a controller, a general purpose processor, or an embedded system. The processor-based module 228 may receive additional inputs from a user through an input device such as a keyboard or a control panel. The processor-based module 228 may also be communicatively coupled to a memory module such as a random access memory (RAM), read only memory (ROM), flash memory, or other type of computer readable memory. Such a memory module may be encoded with a program to instruct the processor-based module 228 to enable a sequence of steps to determine at least one of the components and the concentration of the component 230. In an alternate embodiment, all the components of the exemplary detection system 200 may be incorporated as a single stand-alone module integrated with inspection systems 108, 110 (shown in FIG. 1).

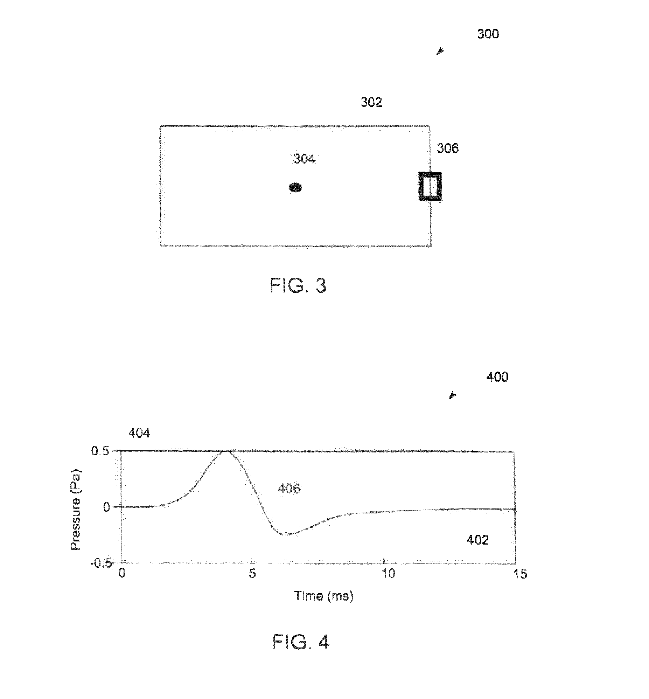

FIG. 3 is a schematic diagram 300 illustrating relative positions of a center of a pressure wave front and a pressure sensor used to measure the acoustic (or pressure) signal in accordance with an exemplary embodiment. In the illustrated embodiment, a rectangle 302 is representative of a chamber and a point 304 is representative of a location in the chamber where the modulated light beam is received by a fluid in the chamber represented by the rectangle 302. Specifically, the point 304 is indicative of the center of the pressure wave front generated in the fluid due to the modulated light beam. In one embodiment, the rectangle 302 may be representative of the first chamber and the point 304 is indicative of the center of a pressure wave representative of the first acoustic signal. In another embodiment, the rectangle 302 may be representative of the second chamber and the point 304 is indicative of the center of a pressure wave representative of the second acoustic signal. In the illustrated embodiment, the pressure sensor 306 is disposed at a position with reference to the rectangle 302. Although, in the illustrated embodiment, the center of the pressure wave is represented as the point 304, in other embodiments, the pressure wave may also be generated along a line created by the modulated light beam along the line to the fluid filled in the chamber.

FIG. 4 illustrates a graph 400 showing variation of a pressure wave front in accordance with an exemplary embodiment of FIG. 3. The x-axis 402 of the plot graph is representative of the time in microseconds and y-axis 404 of the graph 400 is representative of pressure in Pascal. The waveform 406 is representative of a pressure wave detected by the pressure sensor.

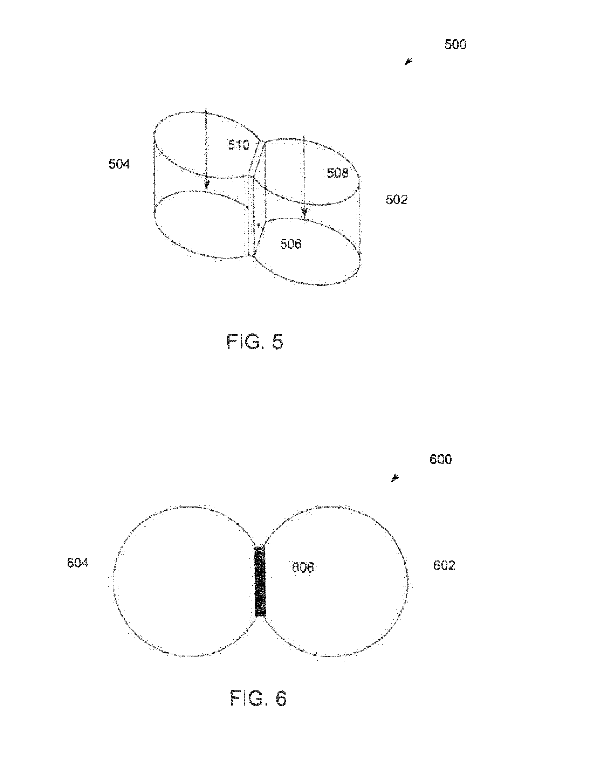

FIG. 5 illustrates a side-by-side arrangement of a detection system 500 in accordance with an exemplary embodiment. In the illustrated embodiment, a first chamber 502 is coupled to a second chamber 504 and disposed side-by-side. A pressure sensor 506 is disposed along a vertical plane between the first chamber 502 and the second chamber 504. The modulated light beams 508, 510 are emitted along a vertical direction into the first and second chambers 502, 504 respectively. In another embodiment, the modulated light beams 508, 510 may be emitted along a horizontal direction and the pressure sensor 506 may be disposed along a horizontal plane between the first chamber 502 and the second chamber 504.

FIG. 6 illustrates a top view of an exemplary detection system 600. In the illustrated embodiment, the detection system 600 includes a circular first chamber 602 coupled to a circular second chamber 604. The pressure sensor 606 is disposed between the circular first chamber 602 and the circular second chamber 604. The embodiments discussed herein are not restrictive and the first chamber 602 and the second chamber 604 may be disposed in any other manner so as to substantially remove the common signal (also called 'common mode signal') between the first acoustic signal and the second acoustic signal. While the embodiments discussed herein depict only two chambers, exemplary techniques are applicable to systems with more than two chambers. The use of multiple chambers can provide for redundancy as well as detection of multiple components.

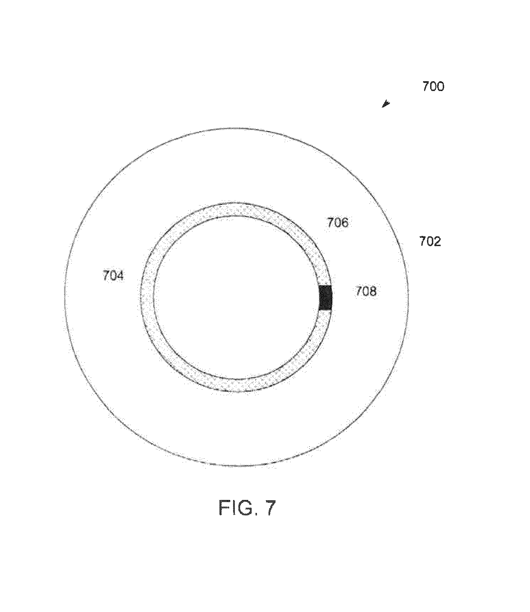

FIG. 7 illustrates a top view of a concentric arrangement of a detection system 700 in accordance with an exemplary embodiment. The detection system 700 includes a first chamber 702 and a second chamber 704 disposed concentrically within the first chamber 702. Both the first chamber 702 and the second chamber 704 are cylindrical shaped. In the illustrated embodiment, a diaphragm 706 is disposed between the first chamber 702 and the second chamber 704. A pressure sensor 708 is disposed within the diaphragm 706. Alternatively, the diaphragm 706 itself may be the pressure sensor.

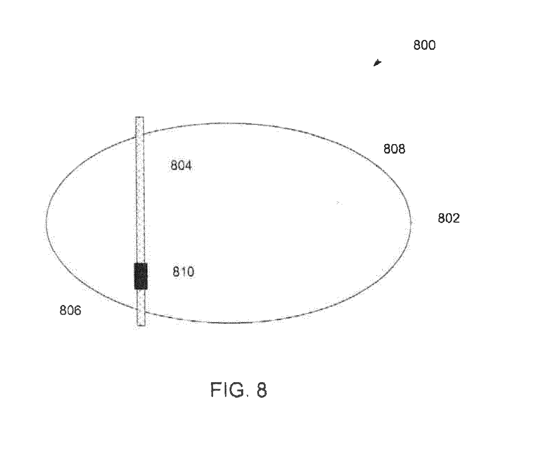

FIG. 8 illustrates another exemplary embodiment of a detection system 800. The detection system 800 includes single enclosure 802 partitioned by a diaphragm 804 to form a first chamber 806 and a second chamber 808. The diaphragm 804 includes a pressure sensor 810 for measuring a difference between a first pressure wave generated in the first chamber 806 and a second pressure wave generated in the second chamber 808. In another embodiment diaphragm 804 itself may be the pressure sensor.

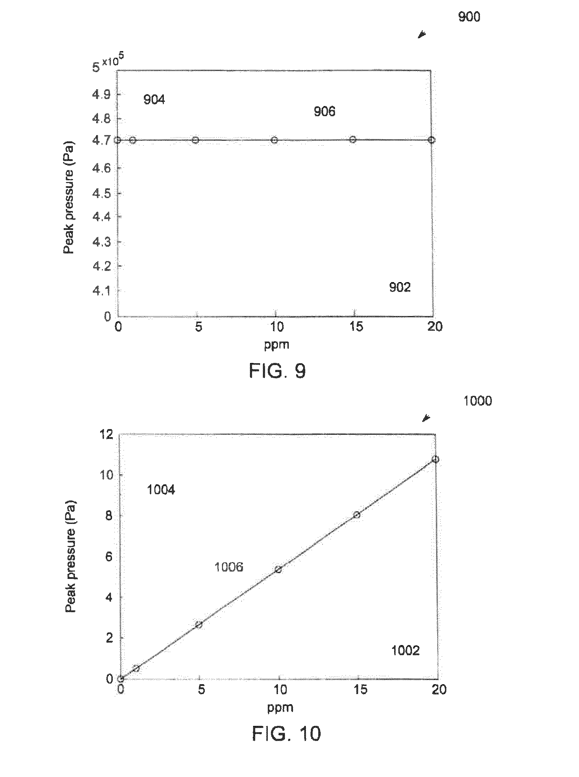

FIG. 9 illustrates a graph 900 showing variation of an amplitude of a photo acoustic pressure wave corresponding to the reference liquid in accordance with an exemplary embodiment. In the graph 900, the x-axis 902 is representative of a concentration in ppm (parts per million) and the y-axis 904 is representative of a peak pressure in Pascal. The curve 906 is representative of the amplitude of the photo acoustic pressure wave generated due to the reference liquid. It may be noted herein that the amplitude value of 4.7×10⁵ Pa is higher compared to the amplitude of the photo acoustic pressure wave generated due to the component dissolved in the sample liquid in accordance with the embodiment shown in the subsequent FIG. 10.

FIG. 10 illustrates a graph 1000 representative of an amplitude of a photo acoustic pressure wave corresponding to the component dissolved in the sample liquid in accordance with an exemplary embodiment. In the graph 1000, the x-axis 1002 is representative of concentration in ppm (parts per million) and the y-axis 1004 is representative of peak pressure in Pascal. The curve 1006 is representative of the amplitude of the photo acoustic pressure wave generated due to the component dissolved in the sample liquid. In the illustrated embodiment, the amplitude value of the curve 1006 is in the range of 0Pa-12Pa which is smaller compared to the amplitude value of the reference liquid illustrated in FIG. 9.

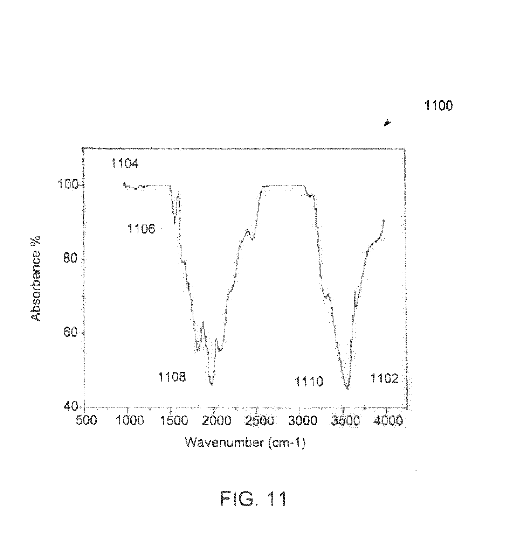

FIG. 11 illustrates a graph 1100 representative of an absorption spectrum corresponding to an insulation oil (with a 0.5mm of path length) of a transformer system, for example, in accordance with an exemplary embodiment. The x-axis 1102 of the graph 1100 is representative of wavenumber (indicated in in cm⁻¹) and the y-axis 1104 of the graph 1100 is representative of an absorbance in percentage values. The curve 1106 is representative of the absorption spectrum of the insulation oil having a minimum absorbance value of about 50% at wavenumbers 2000 cm⁻¹ and 3500 cm⁻¹ represented by numerals 1108, 1110 respectively.

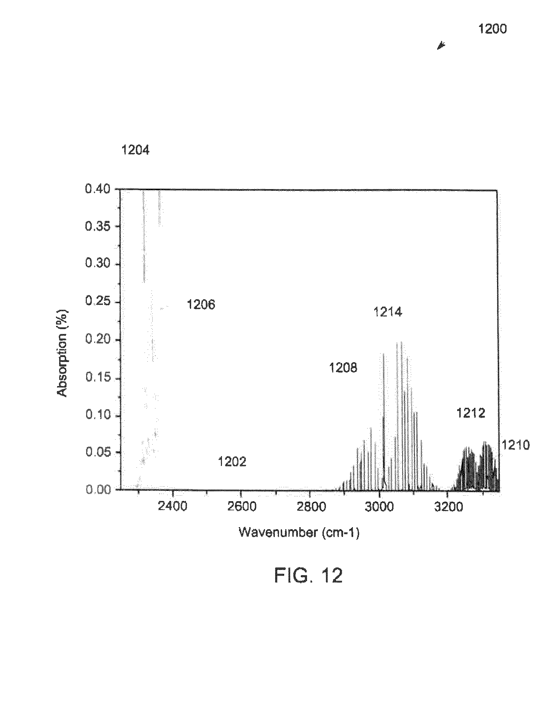

FIG. 12 illustrates a graph 1200 representative of absorption spectra corresponding to a plurality of gaseous components (having 500ppm, and 1mm path length) in accordance with an exemplary embodiment. The x-axis 1202 of the graph 1200 is representative of wavenumber (in cm⁻¹) and the y-axis 1204 of the graph 1200 is representative of an absorbance in percentage values. The curve 1206 is representative of the absorption spectrum of carbon dioxide, the curve 1208 is representative of an absorption spectrum of methane, and the curve 1210 is representative of an absorption spectrum of acetylene. In the graph 1200, acetylene exhibits a peak absorbance value of 0.05% corresponding to a wavenumber of 3300 cm⁻¹ represented by reference numeral 1212, methane exhibits a peak absorbance value of 0.2% corresponding to a wavenumber of 3100 cm⁻¹ represented by reference numeral 1214, and carbon dioxide exhibits a peak absorbance value of 1.4% (not shown in the graph) corresponding to wavenumber of 2300 cm⁻¹. It may be noted herein that the peak absorbance values corresponding to gaseous components illustrated in FIG. 12 are lower compared to the peak absorbance value of the insulation oil. It should be noted herein that all the values in the various embodiments discussed herein should not be construed as a limitation of the invention.

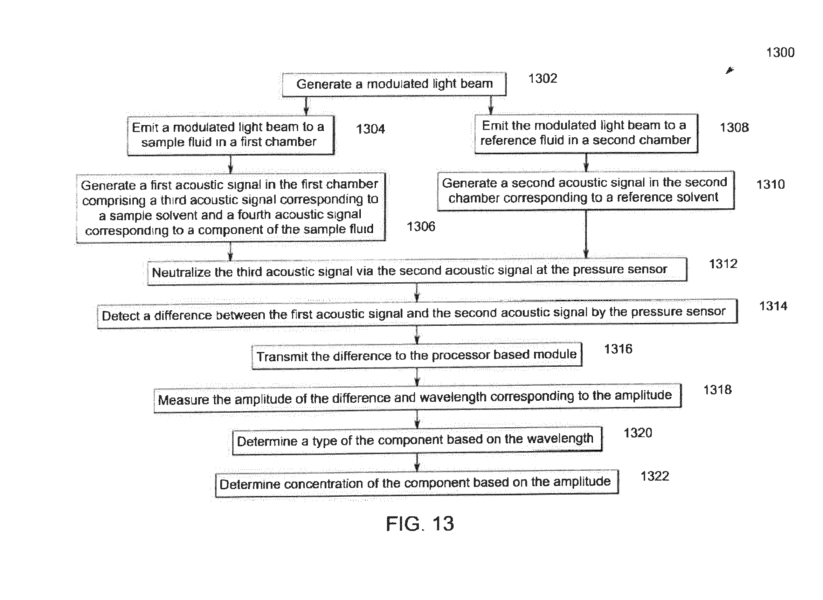

FIG. 13 is a flow chart 1300 illustrating exemplary steps involved in a detection method in accordance with an exemplary embodiment. The method includes generating a modulated light beam 1302 by modulating at least one of an intensity of a light beam 254 and a wavelength of the light beam. The light beam is generated by a light source such as a laser source. The modulated light beam is transmitted to a sample fluid in a first chamber 1304. The sample fluid includes a sample liquid and a component dissolved in the sample liquid. The modulated light beam has a beam wavelength within a range of a spectral absorption wavelength of the component in the sample fluid. In the method discussed herein, in one embodiment, the sample liquid in the sample fluid may be an insulation oil in a transformer system. The emitted modulated light generates a modulated heat flux within the sample fluid. Propagation of modulated heat flux generates pressure waves in the sample fluid. A first acoustic signal is generated 1306 in the first chamber. The first acoustic signal includes a third acoustic signal corresponding to the sample liquid and a fourth acoustic signal corresponding to the component in the sample fluid.

The modulated light beam is also transmitted to a reference fluid in a second chamber 1308. In one embodiment, the reference fluid includes the insulation oil of the transformer system as the reference liquid. A second acoustic signal is generated 1310 in the second chamber and is representative of a pressure signal corresponding to the reference liquid. The first acoustic signal is transmitted to a first side of the pressure sensor disposed between the first chamber and the second chamber. The second acoustic signal is transmitted to a second side opposite to the first side of the pressure sensor. The third acoustic signal corresponding to the sample liquid fully neutralizes or substantially neutralizes the second acoustic signal corresponding to the reference liquid 1312. The pressure sensor detects a difference 1314 between the first acoustic signal and the second acoustic signal.

The signal representative of the difference may include one of an optical signal, an electrical signal, and a pressure signal based on the type of the pressure sensor used. The signal representative of the difference is transmitted from the pressure sensor to a processor-based module 1316. The processor based module measures an amplitude value of the signal representative of the difference. In one embodiment, the measured amplitude value may be a peak value of the signal representative of the difference. In one embodiment, the phase information of the signal representative of the difference may be used to determine the amplitude value.

The processor based module determines the component 1320 based on the range of wavelength of the modulated beam. In some embodiments, a look-up table having data corresponding to the gaseous components and their corresponding absorption spectral range may be used to determine the component. In one example, if the wavelength of the modulated light beam corresponds to a wavenumber in the range of 2200-2400 cm⁻¹, the processor based module determines the component as carbon dioxide. In another example, if the wavelength of the modulated light beam corresponds to a wavenumber in the range of 2900-3100 cm⁻¹, the component is detected as methane. In yet another example, if the wavelength of the modulated light beam corresponds to a wavenumber in the range of 3200-3400 cm⁻¹, the component is detected as acetylene.

In one embodiment, a concentration of the component may be determined 1322 based on the measured amplitude value, using a predetermined calibration chart. In one embodiment, the calibration chart may be determined based on a transfer function. In another embodiment, the calibration chart may be determined based on simulation results. The calibration chart is a look-up table having data entries of concentration values for a range of amplitude values corresponding to each of the gaseous components.

The exemplary systems and methods for inspection enable determination of a concentration of a component in a fluid using photo acoustic spectroscopy (PAS). The technique detects a small amplitude photo acoustic pressure wave corresponding to the component in a relatively large amplitude photo acoustic pressure wave corresponding to the sample liquid. In the case of electrical transformer systems, for example, the exemplary technique performs analysis of dissolved gas without extracting the gaseous components from the insulation oil.

It is to be understood that not necessarily all such objects or advantages described above may be achieved in accordance with any particular embodiment. Thus, for example, those skilled in the art will recognize that the systems and techniques described herein may be embodied or carried out in a manner that achieves or improves one advantage or group of advantages as taught herein without necessarily achieving other objects or advantages as may be taught or suggested herein.

While the technology has been described in detail in connection with only a limited number of embodiments, it should be readily understood that the invention are not limited to such disclosed embodiments. Rather, the technology can be modified to incorporate any number of variations, alterations, substitutions or equivalent arrangements not heretofore described, but which are commensurate with the scope of the claims. Additionally, while various embodiments of the technology have been described, it is to be understood that aspects of the inventions may include only some of the described embodiments. Accordingly, the inventions are not to be seen as limited by the foregoing description, but are only limited by the scope of the appended claims.

Various aspects and embodiments of the present invention are defined by the following numbered clauses:
1. A system comprising:
   a first chamber having a sample fluid;
   a second chamber coupled to the first chamber, wherein the second chamber has a reference fluid;
   a modulated light source for emitting a modulated light beam to the sample fluid and the reference fluid, to generate a first acoustic signal in the first chamber and a second acoustic signal in the second chamber;
   a pressure sensor disposed between the first chamber and the second chamber, for detecting a difference between the first acoustic signal and the second acoustic signal; and
   a processor based module communicatively coupled to the pressure sensor and configured to receive a signal representative of the difference from the pressure sensor and determine at least one of a component and a concentration of the component in the sample fluid based on the signal representative of the difference.
2. The system of clause 1, wherein the sample fluid further comprises a sample liquid, and wherein the component is a gaseous component dissolved in the sample liquid.
3. The system of any preceding clause, wherein the gaseous component comprises acetylene, hydrogen, methane, ethane, ethylene, carbon dioxide, moisture, and carbon monoxide, and combinations thereof.
4. The system of any preceding clause, wherein the reference fluid includes a reference liquid.
5. The system of any preceding clause, wherein the modulated light source comprises a laser source for emitting the modulated light beam having a beam wavelength within a range of a spectral absorption wavelength of the component.
6. The system of any preceding clause, wherein the modulated light source comprises a light source and a modulator device for controlling at least one of an intensity of the light beam, a wavelength of the light beam, and a parameter of the light source.
7. The system of any preceding clause, wherein the pressure sensor comprises a piezo effect based sensor, a cantilever based sensor, a hydrophone, a capacitance based sensor or a membrane based sensor.
8. The system of any preceding clause, wherein the first chamber and the second chamber are disposed in a side-by-side arrangement.
9. The system of any preceding clause, wherein the first chamber and the second chamber are disposed in a concentric arrangement.
10. The system of any preceding clause, wherein the first chamber and the second chamber are separated by a pressure sensor.
11. A method, comprising:
   emitting a modulated light beam to a sample fluid in a first chamber and a reference fluid in a second chamber, wherein the second chamber is coupled to the first chamber;
   generating a first acoustic signal in the first chamber and a second acoustic signal in the second chamber, in response to the emitted modulated light beam;
   detecting a difference between the first acoustic signal and the second acoustic signal via a pressure sensor disposed between the first chamber and the second chamber;
   transmitting a signal representative of the difference from the pressure sensor to a processor based module; and
   determining at least one of a component and a concentration of the component in the sample fluid via the processor based module, based on the signal representative of the difference.
12. The method of any preceding clause, wherein the emitting comprises generating the modulated light beam having a beam wavelength within a range of a spectral absorption wavelength of the component.
13. The method of any preceding clause, wherein the emitting comprises modulating at least one of an intensity of the light beam and a wavelength of the light beam.
14. The method of any preceding clause, wherein generating the first acoustic signal comprises generating a third acoustic signal corresponding to a sample liquid of the sample fluid and a fourth acoustic signal corresponding to the component of the sample fluid.
15. The method of any preceding clause, wherein the second acoustic signal corresponds to the reference fluid comprising a reference liquid.
16. The method of any preceding clause, wherein the detecting comprises neutralizing or substantially neutralizing the third acoustic signal transmitted to a first side of the pressure sensor, via the second acoustic signal transmitted to a second side opposite to the first side of the pressure sensor.
17. The method of any preceding clause, wherein the signal representative of the difference comprises one of an optical signal, an electrical signal, and a pressure signal.
18. The method of any preceding clause, wherein the determining comprises measuring an amplitude value of the signal representative of the difference.
19. The method of any preceding clause, wherein the determining comprises measuring the concentration of the component based on the measured amplitude value.

## Claims

1. A system (200) comprising:
a first chamber (218) having a sample fluid (232);
a second chamber (218) coupled to the first chamber (218), wherein the second chamber (218) has a reference fluid (234);
a modulated light source (202) for emitting a modulated light beam (220) to the sample fluid (232) and the reference fluid (234), to generate a first acoustic signal (238) in the first chamber (218) and a second acoustic signal (240) in the second chamber (218);
a pressure sensor (236) disposed between the first chamber (218) and the second chamber (218), for detecting a difference between the first acoustic signal (238) and the second acoustic signal (240); and
a processor based module (228) communicatively coupled to the pressure sensor (236) and configured to receive a signal representative of the difference from the pressure sensor (236) and determine at least one of a component (230) and a concentration of the component (230) in the sample fluid (232) based on the signal representative of the difference.

2. The system of claim 1, wherein the sample fluid (232) further comprises a sample liquid, and wherein the component (230) is a gaseous component dissolved in the sample liquid, wherein, preferably, the gaseous component comprises acetylene, hydrogen, methane, ethane, ethylene, carbon dioxide, moisture, and carbon monoxide, and combinations thereof.

3. The system of claim 1 or claim 2, wherein the reference fluid (234) includes a reference liquid.

4. The system of claim 1, 2 or 3, wherein the modulated light source (202) comprises a laser source for emitting the modulated light beam (220) having a beam wavelength within a range of a spectral absorption wavelength of the component (230).

5. The system of any preceding claim, wherein the modulated light source (202) comprises a light source (206) and a modulator device (208) for controlling at least one of an intensity of the light beam, a wavelength of the light beam, and a parameter of the light source.

6. The system of any preceding claim, wherein the pressure sensor (236) comprises a piezo effect based sensor, a cantilever based sensor, a hydrophone, a capacitance based sensor or a membrane based sensor.

7. The system of any preceding claim, wherein:
the first chamber (218) and the second chamber (218) are disposed in a side-by-side arrangement, or
the first chamber (218) and the second chamber (218) are disposed in a concentric arrangement.

8. The system of any preceding claim, wherein the first chamber (218) and the second chamber (218) are separated by a pressure sensor (236).

9. A method (1300), comprising:
emitting (1304, 1308) a modulated light beam to a sample fluid in a first chamber and a reference fluid in a second chamber, wherein the second chamber is coupled to the first chamber;
generating (1306, 1310) a first acoustic signal in the first chamber and a second acoustic signal in the second chamber, in response to the emitted modulated light beam;
detecting (1314) a difference between the first acoustic signal and the second acoustic signal via a pressure sensor disposed between the first chamber and the second chamber;
transmitting (1316) a signal representative of the difference from the pressure sensor to a processor based module; and
determining (1320, 1322) at least one of a component and a concentration of the component in the sample fluid via the processor based module, based on the signal representative of the difference.

10. The method of claim 9, wherein the emitting (1304, 1308) comprises generating the modulated light beam having a beam wavelength within a range of a spectral absorption wavelength of the component.

11. The method of claim 9 or claim 10, wherein the emitting (1304, 1308) comprises modulating at least one of an intensity of the light beam and a wavelength of the light beam.

12. The method of claim 9, 10 or 11, wherein generating (1306) the first acoustic signal comprises generating a third acoustic signal corresponding to a sample liquid of the sample fluid and a fourth acoustic signal corresponding to the component of the sample fluid.

13. The method of any one of claims 9 to 12, wherein the second acoustic signal corresponds to the reference fluid comprising a reference liquid, wherein, preferably, the detecting (1314) comprises neutralizing or substantially neutralizing (1312) the third acoustic signal transmitted to a first side of the pressure sensor, via the second acoustic signal transmitted to a second side opposite to the first side of the pressure sensor.

14. The method of any one of claims 9 to 13, wherein the signal representative of the difference comprises one of an optical signal, an electrical signal, and a pressure signal.

15. The method of any one of claims 9 to 14, wherein the determining (1314) comprises measuring an amplitude value of the signal representative of the difference, wherein, preferably, the determining (1314) measuring the concentration of the component based on the measured amplitude value.
